# EUROPEAN PATENT APPLICATION

(11) **EP 4 729 618 A1**
(43) Date of publication of application: **22.04.2026**
(21) Application number: 24826266.9
(22) Date of filing: 20.06.2024
(51) Int. Cl.: C12N 15/79, C12N 15/90, C12N 1/12

(54) **RECOMBINANT VECTOR FOR HIGH EXPRESSION OF GENE**

(30) Priority: 20.06.2023 KR 20230079290
(71) Applicant: CJ Cheiljedang Corporation, Seoul 04560 (KR)
(72) Inventor: RYU, Ae Jin, Seoul 04560 (KR); SHIN, Won Sub, Seoul 04560 (KR); KIM, Ji Young, Seoul 04560 (KR); JANG, Sunghoon, Seoul 04560 (KR)
(74) Representative: Meissner Bolte Partnerschaft mbB
(86) International application number: PCT/KR2024/008526
(87) International publication number: WO 2024/262951

(57) **Abstract**

The present application relates to a DNA construct for high expression of a target gene in a microorganism of the genus *Schizochytrium,* and a method for expressing a target gene in a microorganism of the genus Schizochytrium using same. The DNA construct comprises a 5' homology arm and a 3' homology arm hybridizable with a high-expression site of a microbial genome of the genus *Schizochytrium.*

## Description

### [TECHNICAL FIELD]

### Cross-references with related applications

This application claims a benefit of priority to Korean Patent Application No. 10-2023-0079290, filed on June 20, 2023, the entire disclosure of which is incorporated herein by reference.

This application relates to a DNA construct for high expression of a target gene in a microorganism of the genus *Schizochytrium* and a method for expressing a target gene in a microorganism of the genus *Schizochytrium* using thereof.

### [BACKGROUND ART]

Recently, advances in synthetic biology and metabolic engineering have rapidly facilitated the development and optimization of microbial cell factories by leveraging increasingly sophisticated gene-editing technologies. While such gene-editing technologies are being quickly applied to various microbial cell factories, the development of efficient strains based on these technologies has so far been largely limited to model organisms such as *Escherichia coli, Corynebacterium spp.,* and yeast. Microalgae also lack strain development technology except for some model microalgal species. In particular, in the case of strain development of microalgae in the family *Schizochytrium,* some results on gene transformation have been reported, but there have been no reports on high expression of introduced genes at a breakthrough level.

If the expression level of the introduced gene is not ensured, it may be difficult to clearly confirm phenotypic changes attributable to the introduced gene due to insufficient gene expression. Additionally, in microalgae of *Schizochytrium* family, gene introduction occurs via random insertion into arbitrary sites throughout the genome, and as a result, the expression level of the introduced gene can vary among individual transformants depending on the inserted site. Differences in gene expression levels depending on the chromosomal integration site have been studied in various microorganisms, including *Escherichia coli,* yeast, and microalgae such as *Nannochloropsis.* Based on these studies, strategies involving 'specific insertion of transgenes into loci characterized by high expression' have been developed and successfully applied to achieve high-level transgene expression (Bioresource Technology 340 (2021) 125676).

The "high expression of a gene" should be required during development of *Schizochytrium* strain in order to ensure sufficient gene expression and minimize variation in gene expression among transformants, and thereby achieve more stable expression of transgene and accurate identification of resultant phenotypic changes. The present inventors introduced the green fluorescent protein (GFP) gene into microalgae of *Schizochytrium* and subsequently identified the gene insertion site of GFP in those transformants exhibiting high expression of GFP via genome analysis. In addition, the present inventors have selected the identified gene insertion site as a 'site for high expression of a gene,' and developed a vector composition that can specifically insert a gene into the site based on homologous recombination technology, and finally developed a novel microalga with different characteristics from the parent strain through high expression of various microalgal genes, based on the vector composition.

### [PRIOR ART]

(Non-patent document 1) Ae Jin Ryu, Byeong-ryool Jeong, Nam Kyu Kang, Seungjib Jeon, Min Gi Sohn, Hyo Jin Yun, Jong Min Lim, Seok Won Jeong, Youn-II Park, Won Joong Jeong, Sunghoon Park, Yong Keun Chang, Ki Jun Jeong, Safe-Harboring based novel genetic toolkit for Nannochloropsis salina CCMP1776: Efficient overexpression of transgene via CRISPR/Cas9-Mediated Knock-in at the transcriptional hotspot, Bioresource Technology, Volume 340, 2021, 125676, ISSN 0960-8524

### [DISCLOSURE]

### [TECHNICAL PROBLEM]

An embodiment provides a DNA construct for high expression of a target gene in a microorganism of genus *Schizochytrium.*

The construct is for inserting the target gene into a target region within the genome of the microorganism of genus *Schizochytrium,* and the target region may be a region for high expression within the genome of the microorganism of genus *Schizochytrium.*

More specifically, the DNA construct comprises a 5' homology arm, a target gene, and a 3' homology arm, and each of the 5' homology arm and the 3' homology arm may be hybridizable to the 5' region and the 3' region of the target region (high-expression region), respectively.

In an embodiment, the target region is a region corresponding to a nucleic acid sequence of SEQ ID NO: 1, SEQ ID NO: 2, or a nucleic acid sequence having 95% or more homology thereto in the genome of the microorganism of genus *Schizochytrium,*
the 5' homology arm may be hybridizable to a region located within 20,000 bp upstream from the insertion site of target gene within the target region, and/or
the 3' homology arm may be hybridizable to a region located within 20,000 bp downstream from the insertion site of target gene within the target region.

Another embodiment provides a microorganism of genus *Schizochytrium* comprising the DNA construct.

Other embodiment provides a composition for expressing a target gene and/or a composition for producing a target product in a microorganism of genus *Schizochytrium,* comprising the DNA construct, a microorganism of genus *Schizochytrium,* comprising the DNA construct, or combination thereof.

Other embodiment provides a use of the DNA construct, the microorganism of genus *Schizochytrium* comprising the DNA construct, or a combination thereof, for expressing a target gene and/or for producing a target product in a microorganism of genus *Schizochytrium.*

Other embodiment provides a method for producing a microorganism of genus *Schizochytrium* for use in expression of target gene and/or production of target protein, comprising a step of introducing the DNA construct into the microorganism of genus *Schizochytrium.*

Other embodiment provides a method for expressing a target gene and/or a method for producing a target protein in a microorganism of genus *Schizochytrium,* comprising a step of culturing the microorganism of genus *Schizochytrium* comprising the DNA construct.

### [TECHNICAL SOLUTION]

The present application provides a technology for highly expressing a target gene in a microorganism of genus *Schizochytrium* and/or a technology for producing a target protein in a microorganism of genus *Schizochytrium,* by identifying a region for high expression in the genome of the microorganism of genus *Schizochytrium* and designing a DNA construct capable of introducing a target gene into the high expression region.

### Definition of Terms

In the present application, the phrase "corresponding to" may refer to an amino acid sequence or nucleic acid sequence of a corresponding region identified by aligning the amino acid sequence or nucleic acid sequence of any polypeptide or polynucleotide with a specific amino acid sequence or nucleic acid sequence provided in this application. This sequence alignment may be performed using conventional sequence alignment methods, such as the Needleman-Wunsch algorithm (Needleman and Wunsch, 1970, J. Mol. Biol. 48: 443-453), the Needle program of the EMBOSS package (EMBOSS: The European Molecular Biology Open Software Suite, Rice et al., 2000), Trends Genet. 16: 276-277), etc., but is not limited thereto, and any sequence alignment program known in the art, pairwise sequence comparison algorithm and the like may be appropriately utilized.

In the present application, the sentence "a polynucleotide or a polypeptide comprises or consists of a specific nucleic acid sequence (base sequence) or amino acid sequence" may mean that the polynucleotide or the polypeptide essentially comprises a specific nucleic acid sequence (base sequence) or an amino acid sequence, and may be interpreted as comprising (or not excluding) a "substantially equivalent sequence" in which a mutation (deletion, substitution, modification, and/or addition) is added to the specific nucleic acid sequence (base sequence) or amino acid sequence to the extent that the original function and/or desired function of the polynucleotide or polypeptide is maintained. In one embodiment, a polynucleotide or polypeptide "comprises or consists of a specific nucleic acid sequence (base sequence) or amino acid sequence" may mean that the polynucleotide or polypeptide (i) essentially comprises the specific nucleic acid sequence (base sequence) or amino acid sequence, or (ii) essentially comprises or consists of a nucleic acid sequence or amino acid sequence that has at least 70%, at least 80%, at least 85%, at least 90%, at least 91%, at least 92%, at least 93%, at least 94%, at least 95%, at least 96%, at least 97%, at least 98%, at least 98%, at least 99%, at least 99.5%, or at least 99.9% homology or identity to the specific nucleic acid sequence (base sequence) or amino acid sequence, and maintains its original function and/or desired function. In one embodiment, the desired function may refer to high expression function of gene within the microorganism of genus *Schizochytrium.*

In the present application, "homology" means the percentage of identity between two polynucleotides or polypeptide moieties. The sequence homology from one moiety to another moiety can be determined by known techniques. For example, the homology can be determined by directly aligning sequence information and aligning parameters such as score, identity, and similarity between two polynucleotide molecules or two polypeptide molecules using readily available computer programs. The computer programs may be BLAST (NCBI), CLC Main Workbench (CLC bio), MegAlign^{™} (DNASTAR Inc), etc. In addition, the homology between polynucleotides can be determined by hybridizing polynucleotides under conditions that form a stable duplex between homologous regions, then digesting them with a single-strand-specific nuclease and determining the size of the digested fragments.

In the present application, the terms "homology" or "identity" refer to the degree of similarity between two given amino acid sequences or base sequences, which may be expressed as a percentage. The terms "homology" and "identity" are often used interchangeably.

Sequence homology or identity of conserved polynucleotides or polypeptides is determined by standard alignment algorithms, and may be combined with default gap penalties established by the program being used. In practice, homologous or identical sequences can generally hybridize to all or part of the sequence under moderate or high stringency conditions. It should be appreciated that hybridization also includes hybridization to polynucleotides containing common codons or codons that take codon degeneracy into account.

Whether any two polynucleotide or polypeptide sequences are homologous, similar or identical can be determined using known computer algorithms such as the "FASTA" program using default parameters, for example as in Pearson et al (1988) [Proc. Natl. Acad. Sci. USA 85]: 2444. Alternatively, the Needleman-Wunsch algorithm (Needleman and Wunsch, 1970, J. Mol. Biol. 48: 443-453) as implemented in the Needleman program of the EMBOSS package (EMBOSS: The European Molecular Biology Open Software Suite, Rice et al., 2000, Trends Genet. 16: 276-277) (version 5.0.0 or later) can be determined using the GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego, 1994, and [CARILLO ETA/.](1988) SIAM J Applied Math 48: 1073). For example, homology, similarity, or identity can be determined using BLAST or ClustalW of the National Center for Biotechnology Information Database.

Homology, similarity, or identity of polynucleotides or polypeptides can be determined by comparing sequence information, for example, using a GAP computer program such as that of Needleman et al. (1970), J Mol Biol. 48:443, as disclosed, for example, in Smith and Waterman, Adv. Appl. Math (1981) 2:482. In brief, the GAP program can be defined as the total number of symbols in the shorter of the two sequences divided by the number of similarly arranged symbols (i.e., nucleotides or amino acids). Default parameters for the GAP program include (1) a binary comparison matrix (containing values of 1 for identity and 0 for non-identity) and (2) a comparison matrix as disclosed by Gribskov et al. (1986) Nucl. Acids Res. 48:443, as disclosed by Schwartz and Dayhoff, eds., Atlas Of Protein Sequence And Structure, National Biomedical Research Foundation, pp. 353-358 (1979). 14: 6745 weighted comparison matrix (or EDNAFULL (EMBOSS version of NCBI NUC4.4) permutation matrix); (2) a penalty of 3.0 for each gap and an additional penalty of 0.10 for each symbol in each gap (or a gap opening penalty of 10, a gap extension penalty of 0.5); and (3) no penalty for terminal gaps.

In the present application, "polynucleotide" means a polymer of nucleotides in which nucleotide units (monomers) are covalently bonded to form a long chain, a DNA or RNA strand of a certain length or longer, and more specifically, a polynucleotide fragment encoding the variant.

In the present application, the term "homologous arm or homology arm" refers to a nucleic acid sequence used to insert a gene into a target region, and may refer to, for example, a nucleic acid sequence used for gene insertion by homologous recombination (HR). More specifically, the homology arm may refer to a sequence that can hybridize to the 5'-terminal (or left or upstream) region of the target nucleic acid sequence site (5' homology arm, left arm, or upstream arm) and/or a sequence that can hybridize to the 3'-terminal (or right or downstream) region (3' homology arm, right arm, or downstream arm)). The 5'-terminal (or left or upstream) region and the 3'-terminal (or right or downstream) region may refer to a region located within about 100 kbp toward the 5'-terminal (or left or upstream) side of the target region and a region located within about 100 kbp toward the 3'-terminal (or right or downstream) side, respectively, but are not limited thereto. The 5'-terminal (or left or upstream) arm and the 3'-terminal (or right or downstream) arm may each independently have a length of about 10 bp to about 5,000 bp, but are not limited thereto.

In the present application, the term "capable of hybridization to or hybridizable to" a given nucleic acid sequence region may refer to a state in which a nucleic acid sequence can form a duplex with the nucleic acid sequence, including a complementary sequence to all or part of the nucleic acid sequence region.

In the present application, when the term "hybridization" is used regarding a homologous arm, it may be interpreted as having the same meaning as "homologous recombination," and the two terms may be used interchangeably.

In the present application, the term "about" described before a numerical value may be used to comprehensively refer to a numerical value within a range equivalent to or similar to the numerical value described thereafter. In an embodiment, the equivalent or similar range may refer to, but is not limited to, a range of ±20%, ±15%, ±10%, ±5%, ±3%, ±2%, or ±1% of the described value.

Hereinafter, the present invention will be described in more detail.

An embodiment provides a DNA construct for inserting a target gene into a target region (high-expression region) within the genome of the microorganism of genus *Schizochytrium.*

More specifically, the DNA construct may comprise a 5' homology arm, a target gene, and a 3' homology arm.

In other words, the DNA construct may have the following structure:

5'- [X]- [Y]- [Z]-3' (Formula 1)

In the formula, X represents the 5' homology arm, Y represents the target gene, and Z represents the 3' homology arm.

The DNA construct may be for inserting the target gene into a target region within the genome of the microorganism of genus *Schizochytrium.*

The target region may be a region of high expression of a gene within the genome of the microorganism of genus *Schizochytrium.*

In an embodiment, the target region may be a region corresponding to SEQ ID NO: 1 (Contig 3, 2001468 (Hindlll), SCH_00001433), SEQ ID NO: 2 (Contig 3, 1116023 (Hindlll), SCH_00001298), or a nucleic acid sequence having a homology of at least 60%, at least 65%, at least 70%, at least 75%, at least 80%, at least 85%, at least 90%, at least 95%, at least 96%, at least 97%, at least 98%, at least 98.5%, at least 99%, at least 99.5%, at least 99.6%, at least 99.7%, at least 99.8%, or at least 99.9% thereto in the genome of the microorganism of genus *Schizochytrium.*

The sequences of the target regions of the present application are shown in Table 1 below.

**[Table 1]**

| Explanation | Nucleic acid sequence (5'→3') | SEQ ID NO |
|---|---|---|
| Contig 3, 2001468 (Hindlll), SCH_000014 33, Similar to AIH: Aginatine deaminase (*Arabidopsis thaliana*) | AGTGCACGAGGCCCGATCTCATAACCGGCAATATCATA AATTTGATGTGCAAGCTCTCGTCCAGTCTTGCTATCCGG GTCTTCACAAATATACGATCTAACAAGCTTTTCATATGCT TCTCTGGTTACTTTGGGGGCATGCAGCCCACCATCTTC CTCCGATACCTCCTTTGCTCGCGATGCATTATACTTTTT AGCCCCCTCTTCACTACTCTGAGATTGAGAGCTAAGTC GTTTCGCGTGTTCTGCTTCCTGTTCAGCAATAAATGCTG CTAGTGATTCTTCCTCTTCCCTTGCATACCGCGTCATAC GTTCCTCTTCTGTTTCTTCACCCTGACTACTTTTATCAAG ATCGCCAGGTGTTGTGAAATCGTCATCGCTAGAATCGG ATTGCACACTTACAATATGTGAAGTATTAAGAAGCAACT CACTGTCTCGTTGAATAGCTGCCGCCCCATGCTGAGCA GCAAGACCAATTTGAGACGTGTGCATAACCACCGGACG AGACGACCGCCGCTTTCTTCCTGATGTTGTATTAAACAT GTCAAGAATATCTAAAGACGAATCTAGTTGGGTTTCCCC ATCTTCAATTCGCGCCATGGTCACACGAATATTACCTTT TCGACGTGGATTTGACATAAAAGTCTTTGTGGAATTTGA TGATGCAGAAGATGAAGAGGAGACTGATGAAGCCATAG ACAGACCGCGACCGCTCAGAGTGTCTGGATGAGCATG ACTGCTGCTTTCACCTTCTATACCGGAGTAGCTTGTAGT ACTCAGTGCATCAAGCGCTGCATGACTGCTACTTTCAG CCTCTAATCCGCTATAGCTAGCAATGCTAAGTCCATCG GGCGGGACATAGCTATTGCTTTCGCCGTCAAATCCAAT GTAGCTTGTAATACTAAGCGCATCTGGAGCTACATGACT GACGCTGTCACCTTCATAACCGTGACCGCTAGCATAAC TGCTTTCCTCCTCTTCTGTTCGTGCAAGCATATGACCAA TATTCTGAAACTCCACATGGCCCCTTCTAGACGAAAAGT CATTCAATCTGGATTCCAGGCTCAATGACTCGTGCGCA TCAATGTGCGATGAGGATTGACTTACCTCGTCATATATT TGTGAGTTTGCTGTATCATCCGCATAATCCGGGTCATG CTCACTCGATGTAGCAAGTGCTTCGTCATGATCTGAAG GAATGTCGTCTTCAACTTCAGATCCGTCTGCCTCCCTG CTTGAGATATCAGGGTTCGAGTCTAGGTCATCTTTGCCT ATCGAAGGCGAATTACGAGGCTCTGAACTTGTTTCGAC AGATGCGGTCGGGGTATCATCCTGCTCCATAGTCTCTT GGTCAATTCTGCTGATAGTGCTGTCTCCTTCTTGAGACT GGCCCTGAATATCACTTGATTCTGTACTGCCAAATGTAG | 1 |

| | | |
|---|---|---|
| | ACAAGTTCAGAGAAGGGGTTGAAATGTTCAGAGATCTA ACAGTCCCGAAGTGATTCCCATCGTTTGAAGATGACTC AGACTCAACTCTTGAGAGAAATGTCCCAACGCCACTTC CCCTAGGTTCTTTTGAACCAGCGCTTTCGCCGCTAGCG GTTGACCAGTGGTTTAGGAAGGAAGTCTGCTGATCTGA AGCAGCTAGGAAAATGTTGGCTTCATCTTCATCTTTCGT CACCTTTCCATCATCAGGGTTCTTACCAAGGTGGATGTC TGCCAAGTTAACTTCTGATCCATCCTGGCTAGATTGAAC TGTCTCTAAAGTCTCTTTCGCAATTCCTGACGTGTCTTC AAGTTCTTCATAATCACTTTGCTCGCTAGTTGAAATATTA TCTGCCGAGCTCGCGTCCGAATTCACCATTGGATGTTC TGAAAGAGCTCTTTTAATAGAAATCTCCTCAGGACTACG GTCAGACAGCTCCTGCGCTGCCTCAGCACCATCATCAT TGACACATCTAATGTTGTT | |
| Contig 3, 1116023 (Hindlll), SCH_000012 98, Similar to cyc: Cytochrome *C* (*Lampetra tridentata*) | TAGGCCTTAGGTAGCCAGCCATAGCTCGAGCGTCGCG AGAATCCATCTTTCGATCAAGAACTAAGGCAGGAAAGG AAATGAATGAAGGCCTCATGCGAATGTGCGGTCATCCG CGGAGTCTCTTGCCGCGGAGCAAGCAAGCCTCGCAGG CCTTAAGTGGGGTCCATGGGCGGCGGGCGGCAACGAC TCCGAAATGCTTTGTGAAGAAAGCAAACGCAGGCTAGA TGCTAGATGTGGCGAGAACTCCTTCAGGACCTTGACGC CGAGGTCTTTCTTTCTGCAACAAAGATTGATGTGATAAT TCTCAACTCTGACAAGCGATGCCGCTCTACTTACTTACC TCTGGAGTTACTACTTACTGCTTCTGTCGGGACTCCAAA GCATTACATTTCCATCCTTGGAGACTGTAATGTATCCAT GTAAATCGTGATCGAAGTTGACAAGCAGGCCAAACAAA GCACGAGCCGCAATGCTGCACAACAAACAAACAGATAG ATAGATAGATATCTTGCAGCCTGGGGACCGCTTTGGCA AGCTTTTATGGCTTGAACAAGACATGATCCTGCGTTTAT CTCAAAGAGCCACGCGCAATCTGCACAGGCTGCTTAAC CTCGAAGCAGCGCTGTCGTTGAGACTAATCAATCTCTC GCAACGGGAGAGCTGCTCGGGAGGTTCTTTTCGGATGT GTGGCTCACCTTGCTTGGGGGTTCCTGTAATATCTGCT TCTCAATTAACATTTACTACCACATGTTGGAAACACCCT CACATCTATTACCAGAAAAGGAATTTCCGTCCGCGATAG GTTCACAGACCGCGCAGGTCGTAGCACGCGCAGGAAC CACTATTTTAAAGACTGCGCAACTCGGTCCTGCCTGAAA AGAAATATTTTGTACTCATTAGGAAATACGTAGTCAATTA ATAGCACTAATTTGTATAGGCCTACCTAACAGTAGTACT ATATGAATATTTTTTAAGAATTGAGGTAGTCCTGGCAGT AGACCCTGGAGTTGATGGAGTCATCACCATTCTACTTTT GAGATTCGTCTGCTTTTACCTATAGGTAGTTCGGTATTG TCTTTTCGAAAATGATAATGAGAAAAACACTGTCAGGTT CAACTTAGTTGCTAACTCTTTGCATGACATGTTGTGTTTT GTAATCTATTCTGCGAACTCGAGACTTGAACTAGCAGC CGAGATAGTGTGACAAACTTCATTGGGAAGGACGCAAG | 2 |
| | GTTATCAAATGTATAGCGGGAGGATTGATTTGTGAATTT GTTTACCTTAGAGAGGCTGCCTTTTCTTTAGGTACCACT ACTACCACTTGCAAAGGAACTTACTCCGAGGGACGATG AGAGGTGATGCGTGGTTGGTAGAGGGATTTTTGCGCCT GGCATTGAAACATTTCGCATCAGGTGGTACCTTGTATTG CTGATATACAATGGAGTGATTATTTTGGCTTGAGTCTTT ACCCAAGTTCAAAGACTGATAGTAGTTCTATCTGAGAGC TGATCTGCCTTGACTCGAATTTCTACCCAACTAGTTAGT GAAAACTCATTTGCGTTAATGAGTAGTTGCCACGTAGTA CAGGAGCATGTAGTATGCAAACTTTCAATTCAAATTAGG GGAAAGTTCCTCGGTGAGAGAGTGATTATTACGAGGAA CCAAAGTTCCACCTGCTTACAACTGCACAAATAAGAAGC ACACGGCGTCATACTAGTTTTGCCGAGTTGGTCAAGAG AATTCATTGTAGACTTTCCAACGATGTTATTAGACAAAA CACTATTTTTCTTCTGCTTCAGCAACAGAGAAATGGGTT TGCTTAACTTCCGTGCGGTCTTCTGTCTTGGATGAATAA TCAAAATCTACAGCTTATAAAAATTATTTAAGTGTACGCA AAATGTGGTCTTATCCACTTTCATTTACGTTCTGTGTACT ACGAATAAATAAATTACAGAATTGAATTTGGGTATCTACT AGAGTCAACCACAACGACCACAGTCAAGGTTGCAGACT TTAACATAT | |

The 5' homology arm may be hybridizable (homologous recombination) to the entirety or a portion of a region (for example, a region having a length corresponding to the length of the homology arm and capable of hybridization (homologous recombination) to the homology arm) located within about 100,000 bp (for example, about 1 bp to about 100,000 bp, about 1 bp to about 50,000 bp, about 1 bp to about 10,000 bp, about 1 bp to about 7,500 bp, about 1 bp to about 5,000 bp, about 1 bp to about 2,500 bp, about 1 bp to about 2,000 bp, about 1 bp to about 1,500 bp, about 1 bp to about 1,400 bp, about 1 bp to about 1,300 bp, about 1 bp to about 1,200 bp, about 1 bp to about 1,100 bp, about 1 bp to about 1,000 bp, about 1 bp to about 750 bp, or about 1 bp to about 500 bp) in the 5' direction or upstream of the target region (region for high expression of a gene, more specifically, based on the target gene insertion site within the target region (region for high expression of a gene)). The 5' homology arm may have a length of about 10bp to about 5,000bp, about 10bp to about 4,000bp, about 10bp to about 3,000bp, about 10bp to about 2,000bp, about 10bp to about 1,500bp, about 10bp to about 1,400bp, about 10bp to about 1,300bp, about 10bp to about 1,200bp, about 10bp to about 1,100bp, about 10bp to about 1,000bp, about 20bp to about 5,000bp, about 20bp to about 4,000bp, about 20bp to about 3,000bp, about 20bp to about 2,000bp, about 20bp to about 1,500bp, about 20bp to about 1,400bp, about 20bp to about 1,300bp, about 20bp to about 1,200bp, about 20bp to about 1,100bp, about 20bp to about 1,000bp, about 23bp to about 5,000bp, about 23bp to about 4,000bp, about 23bp to about 3,000bp, about 23bp to about 2,000bp, about 23bp to about 1,500bp, about 23bp to about 1,400bp, about 23bp to about 1,300bp, about 23bp to about 1,200bp, about 23bp to about 1,100bp, about 23bp to about 1,000bp, about 100bp to about 5,000bp, about 100bp to about 4,000bp, about 100bp to about 3,000bp, about 100bp to about 2,000bp, about 100bp to about 1,500bp, about 100bp to about 1,400bp, about 100bp to about 1,300bp, about 100bp to about 1,200bp, about 100bp to about 1,100bp, about 100bp to about 1,000bp, about 250bp to about 5,000bp, about 250bp to about 4,000bp, about 250bp to about 3,000bp, about 250bp to about 2,000bp, about 250bp to about 1,500bp, about 250bp to about 1,400bp, about 250bp to about 1,300bp, about 250bp to about 1,200bp, about 250bp to about 1,100bp, about 250bp to about 1,000bp, about 500bp to about 5,000bp, about 500bp to about 4,000bp, about 500bp to about 3,000bp, about 500bp to about 2,000bp, about 500bp to about 1,500bp, about 500bp to about 1,400bp, about 500bp to about 1,300bp, about 500bp to about 1,200bp, about 500bp to about 1,100bp, about 500bp to about 1,000bp, about 750bp to about 5,000bp, about 750bp to about 4,000bp, about 750bp to about 3,000bp, about 750bp to about 2,000bp, about 750bp to about 1,500bp, about 750bp to about 1,400bp, about 750bp to about 1,300bp, about 750bp to about 1,200bp, about 750bp to about 1,100bp, about 750bp to about 1,000bp, about 900bp to about 5,000bp, about 900bp to about 4,000bp, about 900bp to about 3,000bp, about 900bp to about 2,000bp, about 900bp to about 1,500bp, about 900bp to about 1,400bp, about 900bp to about 1,300bp, about 900bp to about 1,200bp, about 900bp to about 1,100bp, or about 900bp to about 1,000bp, but not limited thereto.

In addition, the 3' homology arm may be hybridizable (homologous recombination) to the entirety or a portion of a region (for example, a region having a length corresponding to the length of the homology arm and capable of hybridization (homologous recombination) to the homology arm) located within about 100,000 bp (for example, about 1bp to about 100,000bp, about 1bp to about 50,000bp, about 1bp to about 10,000bp, about 1bp to about 7,500bp, about 1bp to about 5,000bp, about 1bpto about 2,500bp, about 1bp to about 2,000bp, about 1bp to about 1,500bp, about 1bpto about 1,400bp, about 1bp to about 1,300bp, about 1bp to about 1,200bp, about 1bp to about 1,100bp, about 1bp to about 1,000bp, about 1bp to about 750bp, or about 1bp to about 500bp,) in the 3' direction or downstream of the target region (region for high expression of a gene, more specifically, based on the target gene insertion site within the target region (region of high expression of a gene)). The 3' homology arm may have a length of about 10bp to about 5,000bp, about 10bp to about 4,000bp, about 10bp to about 3,000bp, about 10bp to about 2,000bp, about 10bp to about 1,500bp, about 10bp to about 1,400bp, about 10bp to about 1,300bp, about 10bp to about 1,200bp, about 10bp to about 1,100bp, about 10bp to about 1,000bp, about 20bp to about 5,000bp, about 20bp to about 4,000bp, about 20bp to about 3,000bp, about 20bp to about 2,000bp, about 20bp to about 1,500bp, about 20bp to about 1,400bp, about 20bp to about 1,300bp, about 20bp to about 1,200bp, about 20bp to about 1,100bp, about 20bp to about 1,000bp, about 23bp to about 5,000bp, about 23bp to about 4,000bp, about 23bp to about 3,000bp, about 23bp to about 2,000bp, about 23bp to about 1,500bp, about 23bp to about 1,400bp, about 23bp to about 1,300bp, about 23bp to about 1,200bp, about 23bp to about 1,100bp, about 23bp to about 1,000bp, about 100bp to about 5,000bp, about 100bp to about 4,000bp, about 100bp to about 3,000bp, about 100bp to about 2,000bp, about 100bp to about 1,500bp, about 100bp to about 1,400bp, about 100bp to about 1,300bp, about 100bp to about 1,200bp, about 100bp to about 1,100bp, about 100bp to about 1,000bp, about 250bp to about 5,000bp, about 250bp to about 4,000bp, about 250bp to about 3,000bp, about 250bp to about 2,000bp, about 250bp to about 1,500bp, about 250bp to about 1,400bp, about 250bp to about 1,300bp, about 250bp to about 1,200bp, about 250bp to about 1,100bp, about 250bp to about 1,000bp, about 500bp to about 5,000bp, about 500bp to about 4,000bp, about 500bp to about 3,000bp, about 500bp to about 2,000bp, about 500bp to about 1,500bp, about 500bp to about 1,400bp, about 500bp to about 1,300bp, about 500bp to about 1,200bp, about 500bp to about 1,100bp, about 500bp to about 1,000bp, about 750bp to about 5,000bp, about 750bp to about 4,000bp, about 750bp to about 3,000bp, about 750bp to about 2,000bp, about 750bp to about 1,500bp, about 750bp to about 1,400bp, about 750bp to about 1,300bp, about 750bp to about 1,200bp, about 750bp to about 1,100bp, about 750bp to about 1,000bp, about 900bp to about 5,000bp, about 900bp to about 4,000bp, about 900bp to about 3,000bp, about 900bp to about 2,000bp, about 900bp to about 1,500bp, about 900bp to about 1,400bp, about 900bp to about 1,300bp, about 900bp to about 1,200bp, about 900bp to about 1,100bp, or about 900bp to about 1,000bp, but not limited thereto.

The insertion site of the target gene within the target region (region for high expression of a gene) may be any site included in the sequence of SEQ ID NO: 1 or SEQ ID NO: 2 described herein, and a person having ordinary skill in the art to which the present application pertains can appropriately set the insertion site of the target gene in consideration of the purpose, conditions, desired effects, etc. For example, a gene insertion site belonging to an intermediate site within the sequence of SEQ ID NO: 1 or SEQ ID NO: 2 (for example, within a site of 800 bp to 1200 bp in the 3' end direction starting from the 5' end) may be arbitrarily determined, and a vector including an upstream sequence (5' homology arm) and a downstream sequence (3' homology arm) based on the site may be produced and used, but is not limited thereto.

The target gene refers to a gene to be inserted into the genome of the microorganism of genus *Schizochytrium,* and may be a gene to be expressed in the microorganism of genus *Schizochytrium* and/or a coding gene for a protein (target protein) to be produced in the microorganism of genus *Schizochytrium.* In the DNA construct provided in the present application, the target gene may be one or two or more (e.g., 2, 3, 4, 5, 6, 7, 8, 9 or 10).

In an embodiment, the target gene may be a coding gene for a target protein, a non-coding sequence such as a regulatory sequence (e.g., a transcription factor, etc.), or a combination thereof.

In an embodiment, the target protein may mean any protein that can be usefully used in various industrial fields such as medicines, pharmaceuticals, foods, cosmetics, agriculture, feed, and fertilizer. In one embodiment, the target protein may be at least one selected from the group consisting of antibodies (e.g., mammalian (e.g., human) derived immunoglobulins (IgG (e.g., IgG1, IgG2, IgG3, IgG4, etc.), IgA, IgD, IgM, IgE, etc.)), a part of antibody (e.g., heavy chain variable region and/or light chain variable region, heavy chain and/or light chain, Fc, CH3, CH2-CH3, hinge, etc.), antibody fragments (e.g., Fab fragments, F(ab)2 fragments, Fv, scFv, scFv-Fc, etc.), antibody analogs (e.g., multibodies comprising multiple antigen-binding domains (e.g., diabody, triabody, tetrabody, etc.), single domain antibodies, affibodies, etc.), receptors, growth factors, enzymes, hormones, transport (or efflux) proteins, fluorescent proteins, etc.), but not limited thereto.

In consideration of the genome size of the host cell (microorganism of genus *Schizochytrium*), the target gene may have a size of about total 30 to 100,000 bp, 30 to 75,000 bp, 30 to 5,000 bp, 30 to 25,000 bp, 30 to 20,000 bp, 30 to 15,000 bp, 30 to 10,000 bp, 30 to 7,500 bp, 30 to 5,000 bp, 30 to 2,500 bp, 30 to 1,000 bp, 100 to 100,000 bp, 100 to 75,000 bp, 100 to 5,000 bp, 100 to 25,000 bp, 100 to 20,000 bp, 100 to 15,000 bp, 100 to 10,000 bp, 100 to 7,500 bp, 100 to 5,000 bp, 100 to 2,500 bp, or 100 to 1,000 bp, but not limited thereto.

In an embodiment, the DNA construct may be an expression vector for expressing the target gene in a microorganism of genus *Schizochytrium.*

In an embodiment, when the target gene is a protein coding sequence, the DNA construct may further comprise one or more expression regulatory sequences (transcriptional and/or translational regulatory elements), such as a replication origin, a promoter, an enhancer, a polyadenylation signal, a terminator, a protein transport and/or degradation signal, and the like. In this case, the regulatory elements may be operably linked to the target gene. In another embodiment, the DNA construct may further comprise one or more elements selected from the group consisting of a selection marker, a reporter gene, and the like, to check genome integration.

The selection marker is used to check whether the DNA construct is inserted into the genome of the microorganism of genus *Schizochytrium* or to select a *Schizochytrium* strain into which the DNA construct is inserted, and can be appropriately selected and used among all selection markers available for selection of *Schizochytrium* strains. For example, markers that confer selectable phenotypes such as resistance to antimicrobial agents (antibiotics), nutrient requirements (metabolism-related enzymes), resistance to cytotoxic agents, or expression of surface variant proteins can be used, but are not limited thereto. For example, the selection marker may be at least one selected from the group consisting of an ampicillin resistance gene, a tetracycline resistance gene, a kanamycin resistance gene, a chloroamphenicol resistance gene, a streptomycin resistance gene, a neomycin resistance gene, a blasticidin resistance gene, a zeocin resistance gene, a hygromycin resistance gene, a puromycin resistance gene, a paromomycin resistance gene, a thymidine kinase (TK) gene, a dihydrofolate reductase (DHFR) gene, a glutamine synthetase (GS) gene, and the like, but is not limited thereto. Examples of the reporter gene include, but are not limited to, one or more selected from the group consisting of fluorescent proteins such as green fluorescent protein (GFP), yellow fluorescent protein (YFP), cyan fluorescent protein (CFP), and luciferase.

Insertion of the DNA construct into the genome of the microorganism of genus *Schizochytrium* may be achieved by one or more selected from the group consisting of homologous recombination (e.g., homology directed repair (HDR)), non-homologous end joining (NHEJ), and the like, but not limited to.

The DNA construct may be for delivery and/or insertion of a target gene into the genome of a microorganism of genus *Schizochytrium* (e.g., into a region for high expression), and/or for expression of the target gene in the microorganism of genus *Schizochytrium.*

In addition, as described above, the DNA construct can insert a target gene into a region for high expression in the genome of the microorganism of genus *Schizochytrium,* and thus can have a function of expressing (e.g., highly expressing) the target gene in the microorganism of genus *Schizochytrium* or producing (e.g., producing at a high level) "a target protein encoded by the target gene and/or a metabolite (intermediate product and/or final product) of a metabolic pathway in which the target protein is involved" (hereinafter, "target product").

Accordingly, the DNA construct may have the following functions:
delivery and/or insertion of a target gene into the genome of a microorganism of genus *Schizochytrium* (e.g., within a target region (region for high expression));
expression of a target gene in a microorganism of genus *Schizochytrium*;
production of a target product in a microorganism of genus *Schizochytrium*;
or a combination of two or more of the foregoing.

Accordingly, another embodiment provides a composition for inserting and/or delivering a target gene into the genome of a microorganism of genus *Schizochytrium* (e.g., within a target region (region for high expression)), and/or a composition for expressing the target gene in the microorganism of genus *Schizochytrium,* comprising the DNA construct described above.

Another embodiment provides a use of the DNA construct for inserting and/or delivering a target gene into the genome of a microorganism of the genus *Schizochytrium* (e.g., within a target region (region for high expression)), and/or for expressing the target gene in the microorganism of the genus *Schizochytrium.*

Other embodiments provide a method for inserting and/or delivering a target gene into the genome of a microorganism of genus *Schizochytrium* (e.g., into a target region (high expression region)), a method for expressing the target gene in the microorganism of genus *Schizochytrium,* and/or a method for producing a microorganism of the genus *Schizochytrium* for use in producing a target product, comprising a step of introducing the DNA construct into the microorganism of genus *Schizochytrium.*

Another embodiment provides a microorganism of genus *Schizochytrium* comprising the DNA construct described above.

In the present application, the microorganism of genus *Schizochytrium* may be at least one selected from the group consisting of *Schizochytrium limacinum, Schizochytrium mangrovei, Schizochytrium aggregatum, Schizochytrium minutum,* etc., but is not limited thereto. More specifically, the microorganism of genus *Schizochytrium* may be at least one selected from the following strains, but is not limited thereto:
*Schizochytrium* sp. CD01-5000 (KCTC 14344BP),
*Schizochytrium* sp. CD01-5004 (KCTC 14345BP),
*Schizochytrium* sp. CD01-1821 (KCTC 14660BP),
*Schizochytrium* sp. CD01-2147 (KCTC 14661BP),
*Schizochytrium* sp. CD01-1003 (KCTC 15201BP),
*Schizochytrium* sp. CD02-8025 (KCTC 15246BP), and
*Schizochytrium* sp. CD03-7004 (KCTC 15006BP).

The microorganism of genus *Schizochytrium* comprising the DNA construct provided in the present application may have increased expression of a target gene contained in the DNA construct and/or production of a target product related to the target gene (a target protein encoded by the target gene and/or a metabolite (intermediate product and/or final product) of a metabolic pathway in which the target protein is involved) compared to a microorganism of genus *Schizochytrium* that does not comprise the DNA construct (e.g., a wild-type microorganism of genus *Schizochytrium* and/or a microorganism of genus *Schizochytrium* that comprises the DNA construct at a region other than the target region).

Another embodiment provides a composition for producing a target product, comprising the DNA construct described above, a microorganism of genus *Schizochytrium* comprising the DNA construct, or a combination thereof.

Another embodiment provides a use of the DNA construct, a microorganism of genus *Schizochytrium* comprising the DNA construct, or a combination thereof, for producing a target product.

Another embodiment provides a method for producing a target product from a microorganism of genus *Schizochytrium,* comprising a step of culturing a microorganism of genus *Schizochytrium* comprising the DNA construct. The method may further comprise a step of preparing a microorganism of genus *Schizochytrium* comprising the DNA construct, prior to the culturing step.

The step of preparing may comprise a step of producing or obtaining a microorganism of genus *Schizochytrium* comprising the DNA construct. In an embodiment, the step of preparing the microorganism of the genus *Schizochytrium* comprising the DNA construct, may comprise a step of introducing the DNA construct into the microorganism of genus *Schizochytrium.* In an embodiment, when the introduction is by an endonuclease system specific for a target region, the step of introducing the DNA construct into the microorganism of genus *Schizochytrium* may further comprise a step of introducing an endonuclease system specific for a target region into the microorganism of genus *Schizochytrium.*

The step of culturing can be performed under typical conditions suitable for culturing the microorganism of genus *Schizochytrium* and/or gene expression in the microorganism of the genus *Schizochytrium.* The culturing means growing the microorganism of genus *Schizochytrium* under appropriately controlled environmental conditions. The culturing can be performed according to appropriate media and culture conditions for the microorganism of genus *Schizochytrium* known in the art, and can be appropriately modified by a person skilled in the art. The cultivation method may comprise, but is not limited to, batch culture, continuous culture, fed-batch culture, or a combination thereof. The medium refers to materials containing nutrients required for culturing the microorganism of genus *Schizochytrium* of the present application as a main component, and may be for supplying nutrients and growth factors including water essential for survival and growth. Specifically, the medium and other culture conditions can be applied without any special limitation as long as they are the medium and culture conditions commonly used for the culture of the microorganism of genus *Schizochytrium,* but in an embodiment, the culturing can be performed while controlling temperature, pH, humidity, atmospheric conditions, etc. in a common medium containing carbon sources, nitrogen sources, phosphorus sources, inorganic compounds, amino acids, and/or vitamins suitable for the culture of the microorganism of genus *Schizochytrium.*

The method for producing the target product may further comprise, after the culturing step, a step of isolating, recovering, and/or purifying the target product. The separation, recovery, and/or purification may be performed under conditions typical for microorganisms of genus *Schizochytrium.*

### [ADVANTAGEOUS EFFECTS]

The novel vector composition of the present invention comprises sequences having homology within the ranges of +20 kbp and -20 kbp from a site for high expression of a gene identified in a *Schizochytrium* strain. The vector for high expression of a gene has a left homologous sequence and a right homologous sequence of the site for high expression of a gene, respectively and has a promoter, target gene, and terminator for the high expression of a gene between the two sequences. The vector for high expression of a gene utilizing the site for high expression of a gene will contribute to the expansion of *Schizochytrium-*based gene expression technology and the acceleration of strain development.

### [BRIEF DESCRIPTION OF THE DRAWING]

Fig. 1 is a graph showing the results of a fluorescence analysis of a *Schizochytrium* transformants including green fluorescent protein.
Fig. 2 is a gel electrophoresis image showing the results of a PCR analysis to determine whether homologous recombination occurs at each site for high expression of a gene.
Fig. 3 is a graph comparing the fluorescence intensities of transformants in which the green fluorescent protein gene was inserted into the site for high expression of a gene (Site 1) based on homologous recombination.
Fig. 4 is a graph comparing the fluorescence intensities of transformants in which the green fluorescent protein gene was inserted into the site for high expression of a gene (Site 2) based on homologous recombination.
Fig. 5 is a cleavage map of pUC19-sfGFP-Zeo.
Fig. 6 is a cleavage map of pUC19-HR(site1)-GsfGFPZeoG-HR(Site1).

### [MODE FOR INVENTION]

Hereinafter, this application will be described in more detail through examples and experimental examples. However, these examples and experimental examples are intended to exemplify this application and the scope of this application is not limited to these examples and experimental examples.

### Example 1: Identification of a site for high expression of a gene in Schizochytrium microalgae

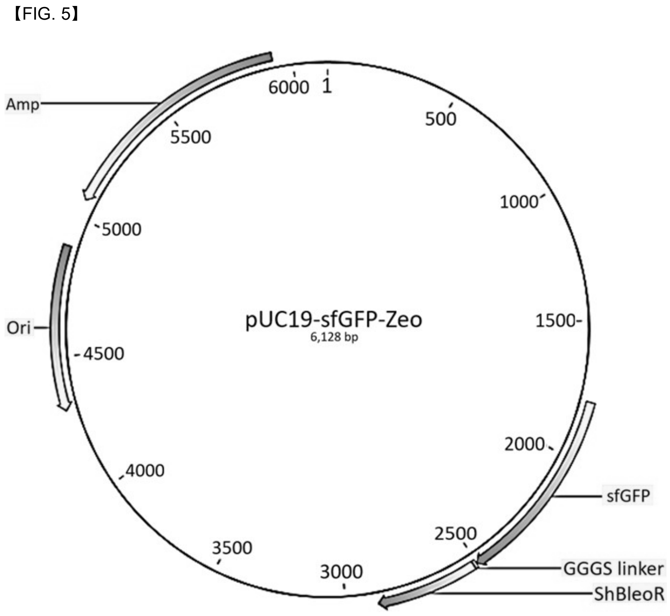

To identify the site for high expression of a gene in *Schizochytrium* microalgae, a gene encoding green fluorescent protein was inserted into pUC19 (Plasmid #50005, Addgene) to produce a vector composition (pUC19-sfGFP-Zeo) expressing green fluorescent protein (see Fig. 5), which was then transformed into the microalgae.

The nucleic acid sequence (SEQ ID NO: 3) of gene coding the green fluorescent protein (GFP) is as follows:

cgtgggggcgggagttcgccctgcgcgacccggccggcaactgcgtgcacttcgtggccgaggagcag

gac

A 250 mL flask was filled with 50 mL of GYPS medium (Glucose 20 g/L, Peptone 6 g/L, Yeast extract 2 g/L, Sea Salt 12.5 g/L, Sucrose 17.1 g/L), and the microalgae of *Schizochytrium* sp. CD01-2147 (KCTC 14661BP) was inoculated and cultured for 48 hours. The cultured microalgae were transferred to two EP tubes (1 mL each), and centrifuged for 1 minute. After removing the supernatant of the centrifuged microalgae culture, the separated microalgae sediment was dissolved in 1X BSS solution (10 mM KCI, 10 mM NaCl, and 3 mM CaCl₂) and centrifuged. After removing the supernatant from the centrifuged sample, the microalgae sediment was dissolved in 50 mM sucrose and centrifuged. This process was repeated three times. After removing the supernatant, 0.1 mL of 50 mM sucrose was added to the microalgae sediment to dissolve it.

The prepared microalgae were transformed with the expression vector of green fluorescent protein (Fig. 5) using electroporation, producing a total of 105 *Schizochytrium* transformants harboring the green fluorescent protein gene. These transformants, in which the green fluorescent protein gene was inserted into any genetic locus, can exhibit varying expression levels of fluorescence.

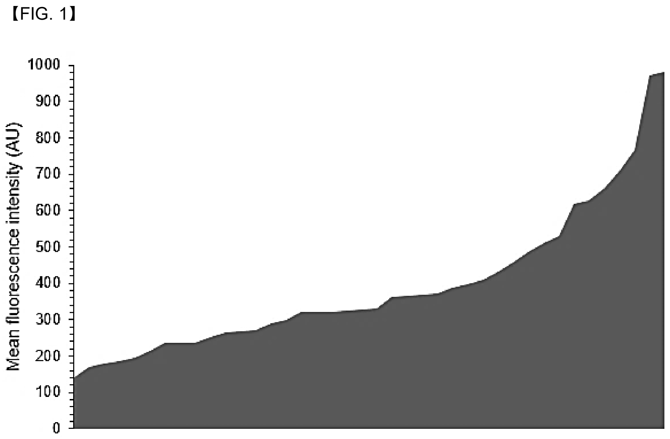

To analyze the GFP expression levels of the 105 tested transformants, the fluorescence intensity of each transformant was measured and compared. More specifically, a flow cytometer was used to detect the 509 nm wavelength which was the fluorescence emission wavelength of green fluorescent protein. The fluorescence intensity emitted near this wavelength was measured for each transformant, to show the results in Fig. 1.

Based on the fluorescence intensity measurements, the top six transformants exhibiting the highest fluorescence intensity were selected.

By analyzing the gene insertion sites of the top six transformants with high fluorescence expression levels, the sites for high expression of a gene were finally identified.

To this end, through genetic analysis of the six transformants selected above, it was confirmed that the exogenous gene (green fluorescent protein gene) was inserted into the same site, Contig 3, 2001468 (SEQ ID NO: 1) (Hindlll, SCH_00001433, Similar to AIH: Aginatine deaminase (*Arabidopsis thaliana*)) (hereinafter, the corresponding site for high expression of a gene is designated as site 1), in five of the six transformants, and into Contig 3, 1116023 (SEQ ID NO: 2) (Hindlll, SCH_00001298, Similar to cyc: Cytochrome C (*Lampetra tridentata*)) (hereinafter, the corresponding site for high expression of a gene is designated as site 2).

### Example 2: High expression of exogenous gene in Schizochytrium microalgae by homologous recombination into sites for high expression of a gene

The feasibility of high expression of a gene using the **sites for high expression of a gene** identified in Example 1 was verified through homologous recombination-based transformation. The green fluorescent protein gene was inserted into Site 1 (SEQ ID NO: 1) and Site 2 (SEQ ID NO: 2) identified in Example 1, using homologous recombination or random insertion.

The vector for homologous recombination was designed as follows: Based on the insertion site within the **sites for high expression of a gene,** Site 1 or Site 2, sequences ranging in length from 800 to 1,500 bp on both sides were amplified to secure arm sequences for homologous recombination.

The representative arm sequences used in this example are listed in Table 2:

**[Table 2]**

| | | Nucleic acid sequence (5' to 3') | SE Q ID NO |
|---|---|---|---|
| Site 1 | 5' homo logy arm | AGTGCACGAGGCCCGATCTCATAACCGGCAATATCATAAA TTTGATGTGCAAGCTCTCGTCCAGTCTTGCTATCCGGGTC TTCACAAATATACGATCTAACAAGCTTTTCATATGCTTCTCT GGTTACTTTGGGGGCATGCAGCCCACCATCTTCCTCCGA TACCTCCTTTGCTCGCGATGCATTATACTTTTTAGCCCCCT CTTCACTACTCTGAGATTGAGAGCTAAGTCGTTTCGCGTG TTCTGCTTCCTGTTCAGCAATAAATGCTGCTAGTGATTCTT CCTCTTCCCTTGCATACCGCGTCATACGTTCCTCTTCTGT TTCTTCACCCTGACTACTTTTATCAAGATCGCCAGGTGTT GTGAAATCGTCATCGCTAGAATCGGATTGCACACTTACAA TATGTGAAGTATTAAGAAGCAACTCACTGTCTCGTTGAATA GCTGCCGCCCCATGCTGAGCAGCAAGACCAATTTGAGAC GTGTGCATAACCACCGGACGAGACGACCGCCGCTTTCTT CCTGATGTTGTATTAAACATGTCAAGAATATCTAAAGACGA ATCTAGTTGGGTTTCCCCATCTTCAATTCGCGCCATGGTC ACACGAATATTACCTTTTCGACGTGGATTTGACATAAAAGT CTTTGTGGAATTTGATGATGCAGAAGATGAAGAGGAGACT GATGAAGCCATAGACAGACCGCGACCGCTCAGAGTGTCT GGATGAGCATGACTGCTGCTTTCACCTTCTATACCGGAGT AGCTTGTAGTACTCAGTGCATCAAGCGCTGCATGACTGCT ACTTTCAGCCTCTAATCCGCTATAGCTAGCAATGCTAAGTC CATCGGGCGGGACATAGCTATTGCTTTCGCCGTCAAATCC AATGTAGCTTGTAATACTAAGCGCATCTGGAGCTACATGA CTGACGCTGTCACCTTCATAACCGTGACCGCTAGCATAAC TGCTTTCCTCCTCTTCTGTTCGTGCAAGCATATGAC | 4 |
| | 3' homo logy arm | CAATATTCTGAAACTCCACATGGCCCCTTCTAGACGAAAA GTCATTCAATCTGGATTCCAGGCTCAATGACTCGTGCGCA TCAATGTGCGATGAGGATTGACTTACCTCGTCATATATTTG TGAGTTTGCTGTATCATCCGCATAATCCGGGTCATGCTCA CTCGATGTAGCAAGTGCTTCGTCATGATCTGAAGGAATGT CGTCTTCAACTTCAGATCCGTCTGCCTCCCTGCTTGAGAT ATCAGGGTTCGAGTCTAGGTCATCTTTGCCTATCGAAGGC GAATTACGAGGCTCTGAACTTGTTTCGACAGATGCGGTC GGGGTATCATCCTGCTCCATAGTCTCTTGGTCAATTCTGC TGATAGTGCTGTCTCCTTCTTGAGACTGGCCCTGAATATC ACTTGATTCTGTACTGCCAAATGTAGACAAGTTCAGAGAA GGGGTTGAAATGTTCAGAGATCTAACAGTCCCGAAGTGA TTCCCATCGTTTGAAGATGACTCAGACTCAACTCTTGAGA GAAATGTCCCAACGCCACTTCCCCTAGGTTCTTTTGAACC AGCGCTTTCGCCGCTAGCGGTTGACCAGTGGTTTAGGAA GGAAGTCTGCTGATCTGAAGCAGCTAGGAAAATGTTGGC TTCATCTTCATCTTTCGTCACCTTTCCATCATCAGGGTTCT TACCAAGGTGGATGTCTGCCAAGTTAACTTCTGATCCATC CTGGCTAGATTGAACTGTCTCTAAAGTCTCTTTCGCAATT CCTGACGTGTCTTCAAGTTCTTCATAATCACTTTGCTCGC TAGTTGAAATATTATCTGCCGAGCTCGCGTCCGAATTCAC CATTGGATGTTCTGAAAGAGCTCTTTTAATAGAAATCTCCT CAGGACTACGGTCAGACAGCTCCTGCGCTGCCTCAGCA CCATCATCATTGACACATCTAATGTTGTT | 5 |
| Site 2 | 5' homo logy arm | TAGGCCTTAGGTAGCCAGCCATAGCTCGAGCGTCGCGAG AATCCATCTTTCGATCAAGAACTAAGGCAGGAAAGGAAAT GAATGAAGGCCTCATGCGAATGTGCGGTCATCCGCGGAG TCTCTTGCCGCGGAGCAAGCAAGCCTCGCAGGCCTTAA GTGGGGTCCATGGGCGGCGGGCGGCAACGACTCCGAA ATGCTTTGTGAAGAAAGCAAACGCAGGCTAGATGCTAGAT GTGGCGAGAACTCCTTCAGGACCTTGACGCCGAGGTCTT TCTTTCTGCAACAAAGATTGATGTGATAATTCTCAACTCTG ACAAGCGATGCCGCTCTACTTACTTACCTCTGGAGTTACT ACTTACTGCTTCTGTCGGGACTCCAAAGCATTACATTTCC ATCCTTGGAGACTGTAATGTATCCATGTAAATCGTGATCGA AGTTGACAAGCAGGCCAAACAAAGCACGAGCCGCAATG CTGCACAACAAACAAACAGATAGATAGATAGATATCTTGCA GCCTGGGGACCGCTTTGGCAAGCTTTTATGGCTTGAACA AGACATGATCCTGCGTTTATCTCAAAGAGCCACGCGCAAT CTGCACAGGCTGCTTAACCTCGAAGCAGCGCTGTCGTTG AGACTAATCAATCTCTCGCAACGGGAGAGCTGCTCGGGA GGTTCTTTTCGGATGTGTGGCTCACCTTGCTTGGGGGTT CCTGTAATATCTGCTTCTCAATTAACATTTACTACCACATGT TGGAAACACCCTCACATCTATTACCAGAAAAGGAATTTCC GTCCGCGATAGGTTCACAGACCGCGCAGGTCGTAGCAC GCGCAGGAACCACTATTTTAAAGACTGCGCAACTCGGTC | 6 |
| | | CTGCCTGAAAAGAAATATTTTGTACTCATTAGGAAATACGT AGTCAATTAATAGCACTAATTTGTATAGGCCTACCTAACAG TAGTACTATATGAATATTTTTTAAGAATTGAGGTAG | |
| | 3' homo logy arm | TCCTGGCAGTAGACCCTGGAGTTGATGGAGTCATCACCA TTCTACTTTTGAGATTCGTCTGCTTTTACCTATAGGTAGTT CGGTATTGTCTTTTCGAAAATGATAATGAGAAAAACACTGT CAGGTTCAACTTAGTTGCTAACTCTTTGCATGACATGTTG TGTTTTGTAATCTATTCTGCGAACTCGAGACTTGAACTAG CAGCCGAGATAGTGTGACAAACTTCATTGGGAAGGACGC AAGGTTATCAAATGTATAGCGGGAGGATTGATTTGTGAATT TGTTTACCTTAGAGAGGCTGCCTTTTCTTTAGGTACCACT ACTACCACTTGCAAAGGAACTTACTCCGAGGGACGATGA GAGGTGATGCGTGGTTGGTAGAGGGATTTTTGCGCCTGG CATTGAAACATTTCGCATCAGGTGGTACCTTGTATTGCTG ATATACAATGGAGTGATTATTTTGGCTTGAGTCTTTACCCA AGTTCAAAGACTGATAGTAGTTCTATCTGAGAGCTGATCT GCCTTGACTCGAATTTCTACCCAACTAGTTAGTGAAAACT CATTTGCGTTAATGAGTAGTTGCCACGTAGTACAGGAGCA TGTAGTATGCAAACTTTCAATTCAAATTAGGGGAAAGTTCC TCGGTGAGAGAGTGATTATTACGAGGAACCAAAGTTCCA CCTGCTTACAACTGCACAAATAAGAAGCACACGGCGTCAT ACTAGTTTTGCCGAGTTGGTCAAGAGAATTCATTGTAGAC TTTCCAACGATGTTATTAGACAAAACACTATTTTTCTTCTG CTTCAGCAACAGAGAAATGGGTTTGCTTAACTTCCGTGC GGTCTTCTGTCTTGGATGAATAATCAAAATCTACAGCTTAT AAAAATTATTTAAGTGTACGCAAAATGTGGTCTTATCCACT TTCATTTACGTTCTGTGTACTACGAATAAATAAATTACAGAA TTGAATTTGGGTATCTACTAGAGTCAACCACAACGACCAC AGTCAAGGTTGCAGACTTTAACATAT | 7 |

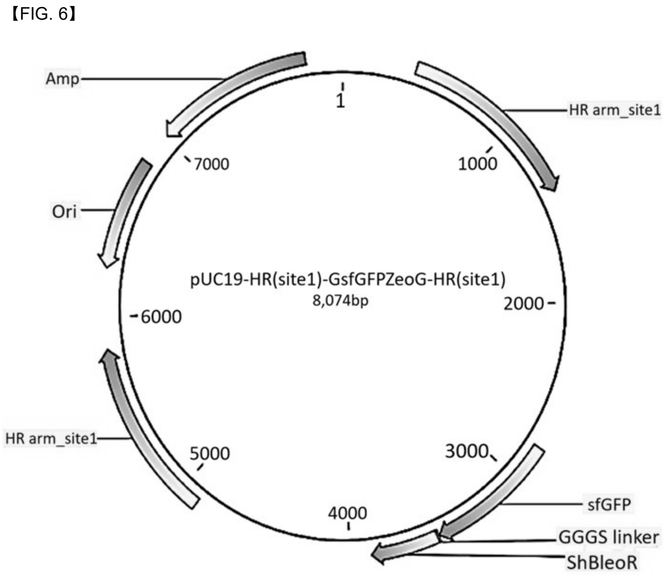

Additionally, the gene coding green fluorescent protein, antibiotic resistance gene expression site, and backbone were amplified from the vector for random insertion used in Example 1 (pUC19-sfGFP-Zeo; Figure 5). The amplified sequences, along with the arm sequences for homologous recombination amplified from Site 1 and Site 2, were cloned using the Gibson assembly method (Gibson Assembly^{®}; NEB), producing homologous recombination vectors targeting Site 1 and Site 2, respectively. To illustrate the structure of the homologous recombination vector, the homologous recombination vector targeting Site 1 (pUC19-HR(site1)-GsfGFPZeoG-HR(site1)) is illustrated in Fig. 6.

The primer sequences used to construct the homologous recombination vectors are described below:
(Primer for amplifying the gene coding green fluorescent protein to the antibiotic resistance gene)
forward primer: CCTCCTCTTCTGTTCGTGCAAGCATATGACGGTACCCTTGATCTTGTG (SEQ ID NO: 8)
reverse primer:
   AGGGGCCATGTGGAGTTTCAGAATATTGGCATGCCTTGAAGC (SEQ ID NO: 9)

### (Backbone)

forward primer:
   ATCATCATTGACACATCTAATGTTGTTAAGCTTGGCGTAATCATG (SEQ ID NO: 10)
reverse primer:
   CCGGTTATGAGATCGGGCCTCGTGCACTGAGCTCGAATTCACTGG (SEQ ID NO: 11)

To construct the vector for random insertion, the sequence from the promoter to the terminator was amplified via PCR from the vector (pUC1 9-sfGFP-Zeo) constructed in Example 1.

Using the prepared vector and the amplified PCR product, transformation of *Schizochytrium* microalgae was performed using electroporation. Transformants were obtained by screening on a medium containing the antibiotic (zeocin).

Genotypic and phenotypic analyses of homologous recombination transformants transformed with the prepared vector for homologous recombination and random insertion transformants transformed with the vector for random insertion were performed. The expression level (fluorescence intensity) was measured and compared according to the presence or absence of gene insertion into a site for high expression of a gene (see Example 1).

Transformants were obtained by inserting green fluorescent protein into a site for high expression of a gene via homologous recombination. Thirty transformants were obtained for each site. A total of 60 transformants were subjected to polymerase chain reaction (PCR)-based genotyping. Two oligomers were designed for PCR: one to selectively bind to the site for high expression of a gene expression site, and the other to the inserted green fluorescent protein gene. PCR was performed using this oligomer pair.

The primer sequences for Site 1 transformation verification are as follows:
Forward primer: TTCCTTCTGAAGAAGGGTCGTTGGTCT (SEQ ID NO:12)
Reverse primer: CCAGGAAAGGACTTGAGATG (SEQ ID NO:13)
The primer sequences for Site 2 transformation verification are as follows:
   Forward primer: GAAAAGGACTTGTGCCTC (SEQ ID NO:14)
   Reverse primer: CCAGGAAAGGACTTGAGATG (SEQ ID NO:15)
   PCR reaction conditions are as follows:
      Tm: 53°C, Cycle: 35, Taq polymerase

Through this PCR, a DNA fragment of about 1500 bp could be obtained when the green fluorescent protein gene was inserted into each site for high expression of a gene (Site 1 and Site 2) through homologous recombination.

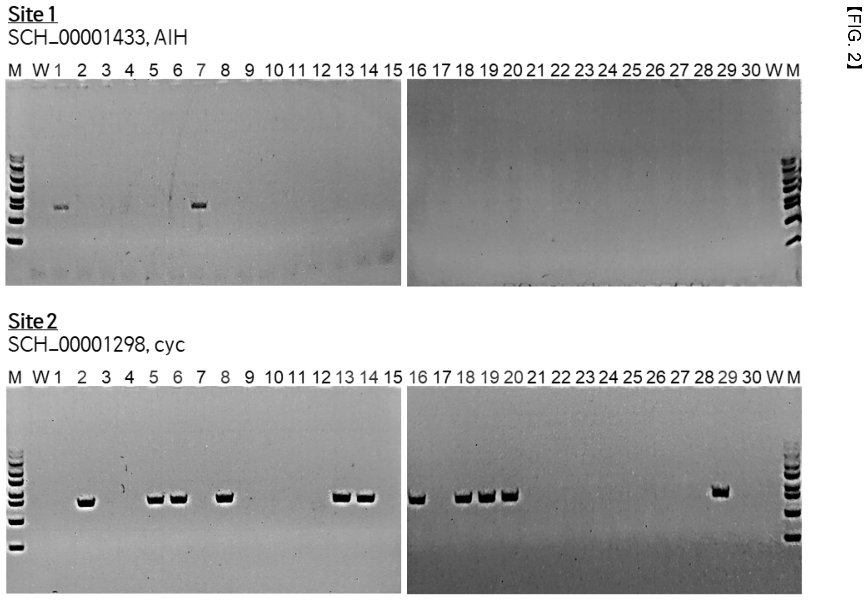

The PCR results are shown in Fig. 2. As shown in Fig. 2, when homologous recombination-based transformation of green fluorescent protein was attempted at Site 1, it was confirmed that the gene was inserted into Site 1 based on homologous recombination in 2 transformants (No. 1 and No. 7) among a total of 30 transformants. In addition, for Site 2, it was confirmed that the gene was inserted based on homologous recombination in 11 transformants (No. 2, 5, 6, 8, 13, 14, 16, 18, 19, 20, and 29) among a total of 30 transformants.

As described above, the expression levels of green fluorescent protein (GFP) in 30 transformants (60 in total) obtained at each site for high expression of a gene through homologous recombination were measured and compared. The expression levels of GFP were measured by inoculating each microalgae *Schizochytrium* transformant into a 250 mL flask containing 50 mL of GYPS medium, culturing for 48 hours, and observing fluorescence intensity. For comparison, a wild-type (WT) strain without a GFP gene insertion was used as a control.

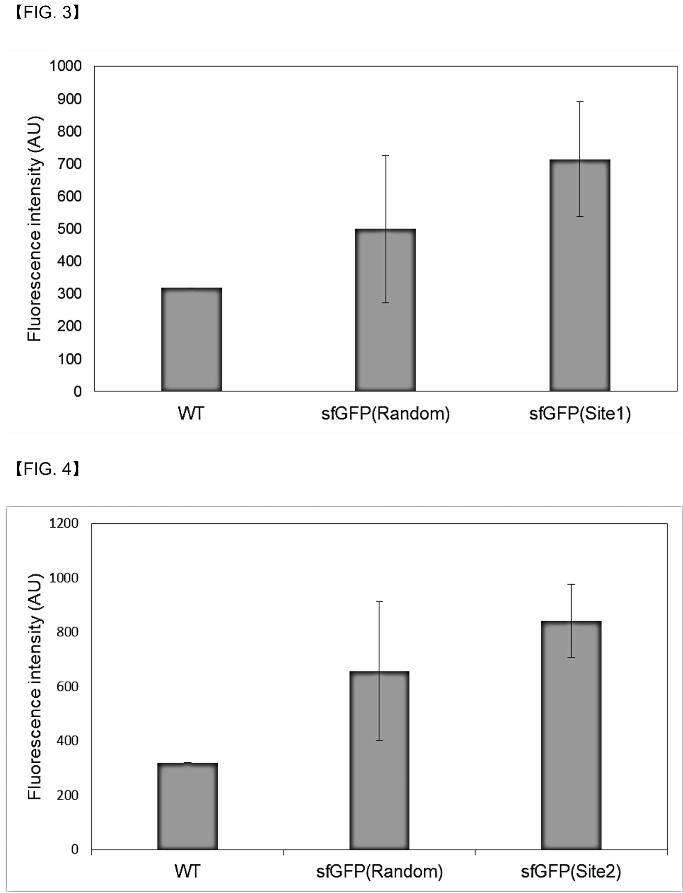

The results are presented in Fig. 3 (fluorescence intensity of a transformant with a GFP gene inserted into a site for high expression of a gene (Site 1)) and Fig. 4 (fluorescence intensity of a transformant with a GFP gene inserted into a site for high expression of a gene (Site 2)).

As shown in FIG. 3, the average fluorescence expression level (AU) of homologous-recombination transformants No. 1 and No. 7 based on the Site 1, a site for high expression of a gene, was 714.61, and the expression level was confirmed to be higher than the average fluorescence expression level (499.51) of 28 transformants in which the gene was randomly inserted into other genomic sites.

In addition, as shown in FIG. 4, the average fluorescence expression level (AU) of a total of 11 homologous-recombination transformants based on the Site 2, a site for high expression of a gene, was 841.54, and the expression level was confirmed to be higher than the average fluorescence expression level (657.05) of 19 transformants in which the gene was randomly inserted into other genomic sites.

In addition, when the transformants were sorted in descending order based on the fluorescence expression level, it was confirmed that the transformants in which the green fluorescent protein gene was specifically inserted into the site for high expression of a gene (Site 1, Site 2) through homologous recombination had an overall superior fluorescence expression level.

### [Accession Number]

Depository authority: Korea Research Institute of Bioscience and Biotechnology, Biological Resource Center (KCTC)
Accession Number: KCTC14344BP
Date of Deposit: 20201026

Depository authority Korea Research Institute of Bioscience and Biotechnology, Biological Resource Center (KCTC)
Accession Number: KCTC14345BP
Date of Deposit: 20201026

Name of deposition authority Korea Research Institute of Bioscience and Biotechnology, Biological Resource Center (KCTC)
Accession Number: KCTC14660BP
Date of Deposit: 20210823

Depository authority Korea Research Institute of Bioscience and Biotechnology, Biological Resource Center (KCTC)
Accession Number: KCTC14661BP
Date of Deposit: 20210823

Depository authority Korea Research Institute of Bioscience and Biotechnology, Biological Resource Center (KCTC)
Accession Number: KCTC15201BP
Date of Deposit: 20221121

Depository authority Korea Research Institute of Bioscience and Biotechnology, Biological Resource Center (KCTC)
Accession Number: KCTC15246BP
Date of Deposit: 20221215

Depository authority Korea Research Institute of Bioscience and Biotechnology, Biological Resource Center (KCTC)
Accession Number: KCTC15006BP
Date of Deposit: 20220620

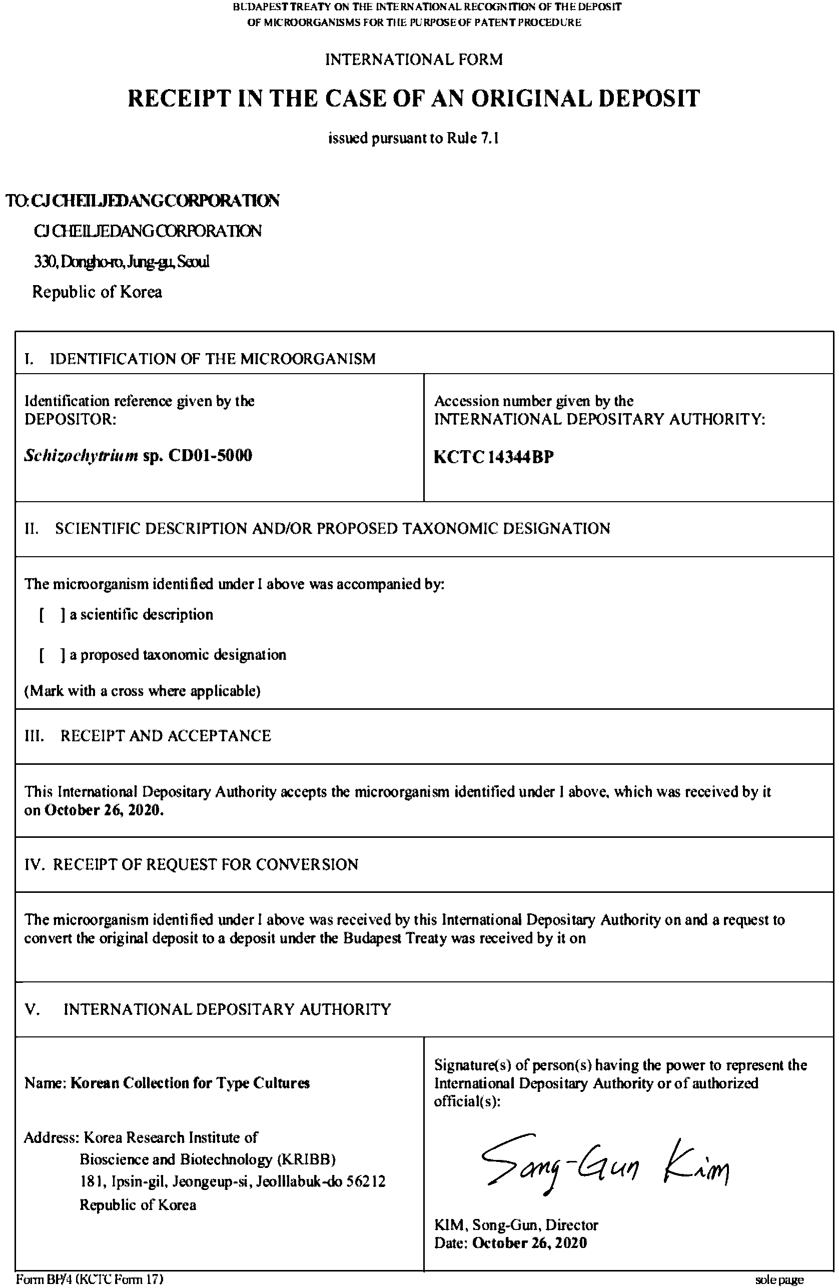
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: CJ CHEILJEDANG CORPORATION
CJ CHEILJEDANG CORPORATION
330, Dongho-ro, Jung-gu, Seoul
Republic of Korea

I. IDENTIFICATION OF THE MICROORGANISM
Identification reference given by the
DEPOSITOR:

Schizochytrium sp. CD01-5000 | Accession number given by the
INTERNATIONAL DEPOSITARY AUTHORITY:

KCTC 14344BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on October 26, 2020.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):


KIM, Song-Gun, Director
Date: October 26, 2020
Form BP/4 (KCTC Form 17) sole page

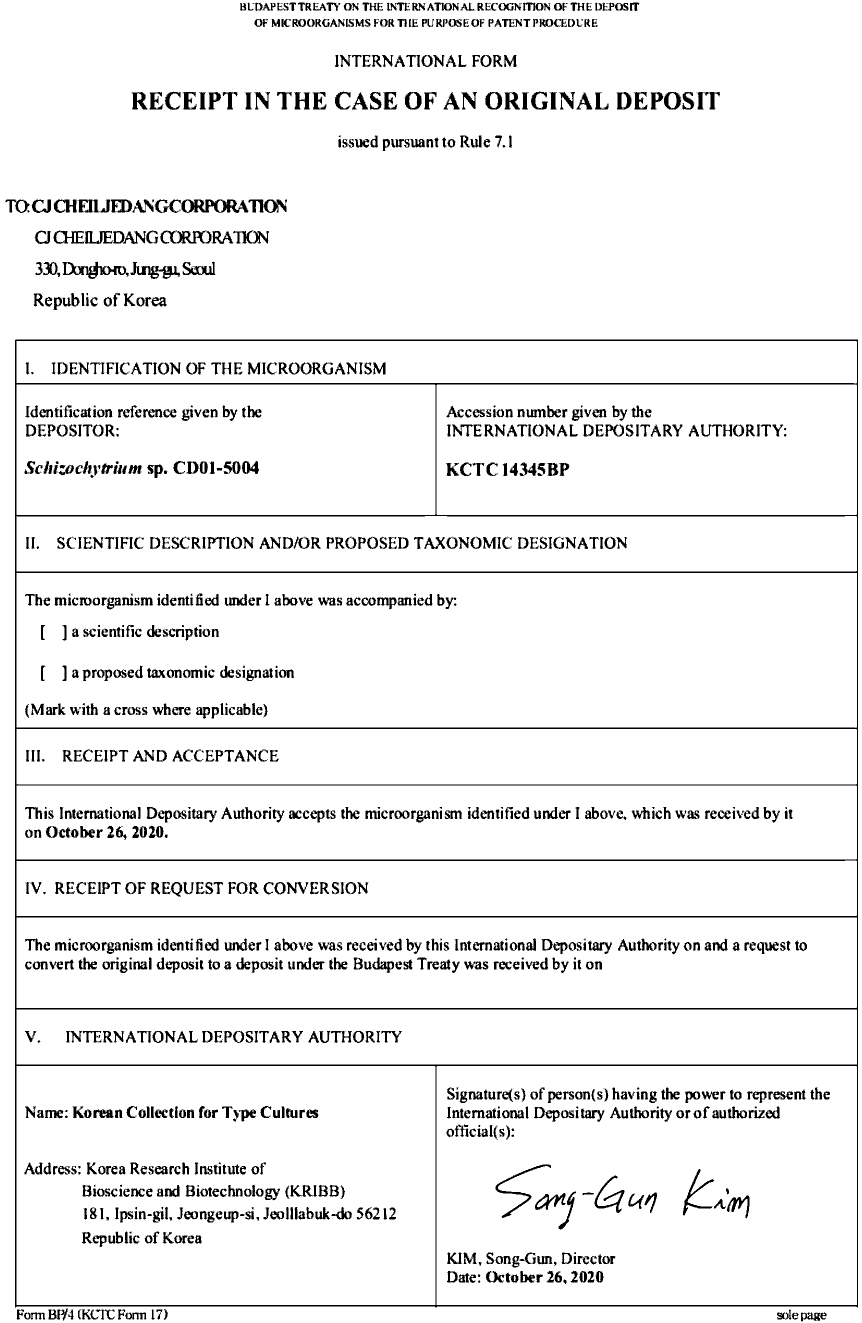
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: CJ CHEILJEDANG CORPORATION
CJ CHEILJEDANG CORPORATION
330, Dongho-ro, Jung-gu, Seoul
Republic of Korea

I. IDENTIFICATION OF THE MICROORGANISM
Identification reference given by the
DEPOSITOR:

Schizochytrium sp. CD01-5004 | Accession number given by the
INTERNATIONAL DEPOSITARY AUTHORITY:

KCTC 14345BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on October 26, 2020.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeolllabuk-do 56212
Republic of Korea | Signature(s) of person(s) having the power to represent the
International Depositary Authority or of authorized
official(s):


KIM, Song-Gun, Director
Date: October 26, 2020
Form BP/4 (KCTC Form 17) sole page

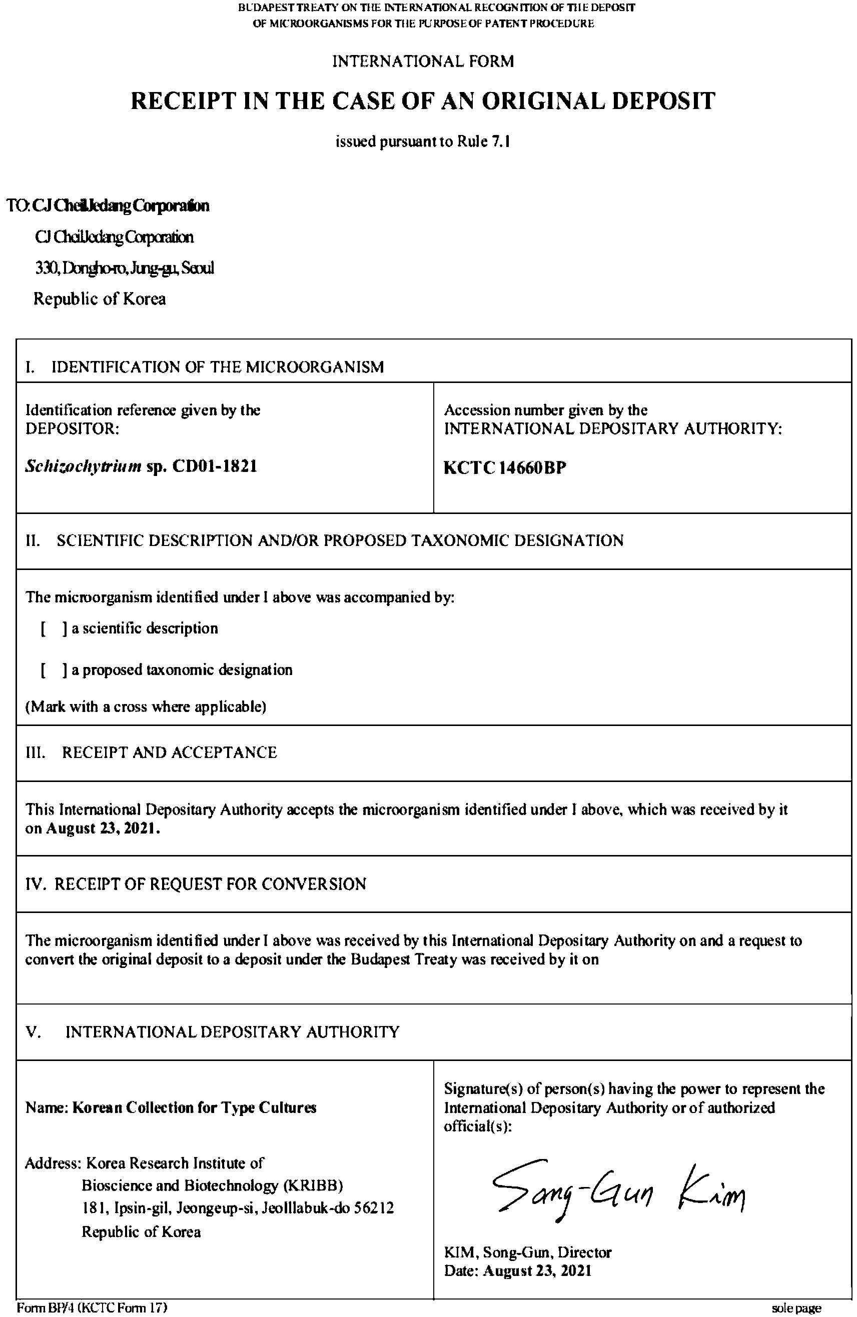
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: CJ CheilJedang Corporation
CJ CheilJedang Corporation
330, Dongho-ro, Jung-gu, Seoul
Republic of Korea

I. IDENTIFICATION OF THE MICROORGANISM
Identification reference given by the
DEPOSITOR:

Schizochytrium sp. CD01-1821 | Accession number given by the
INTERNATIONAL DEPOSITARY AUTHORITY:

KCTC 14660BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on August 23, 2021.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):


KIM, Song-Gun, Director
Date: August 23, 2021

Form BP/4 (KCTC Form 17)
sole page

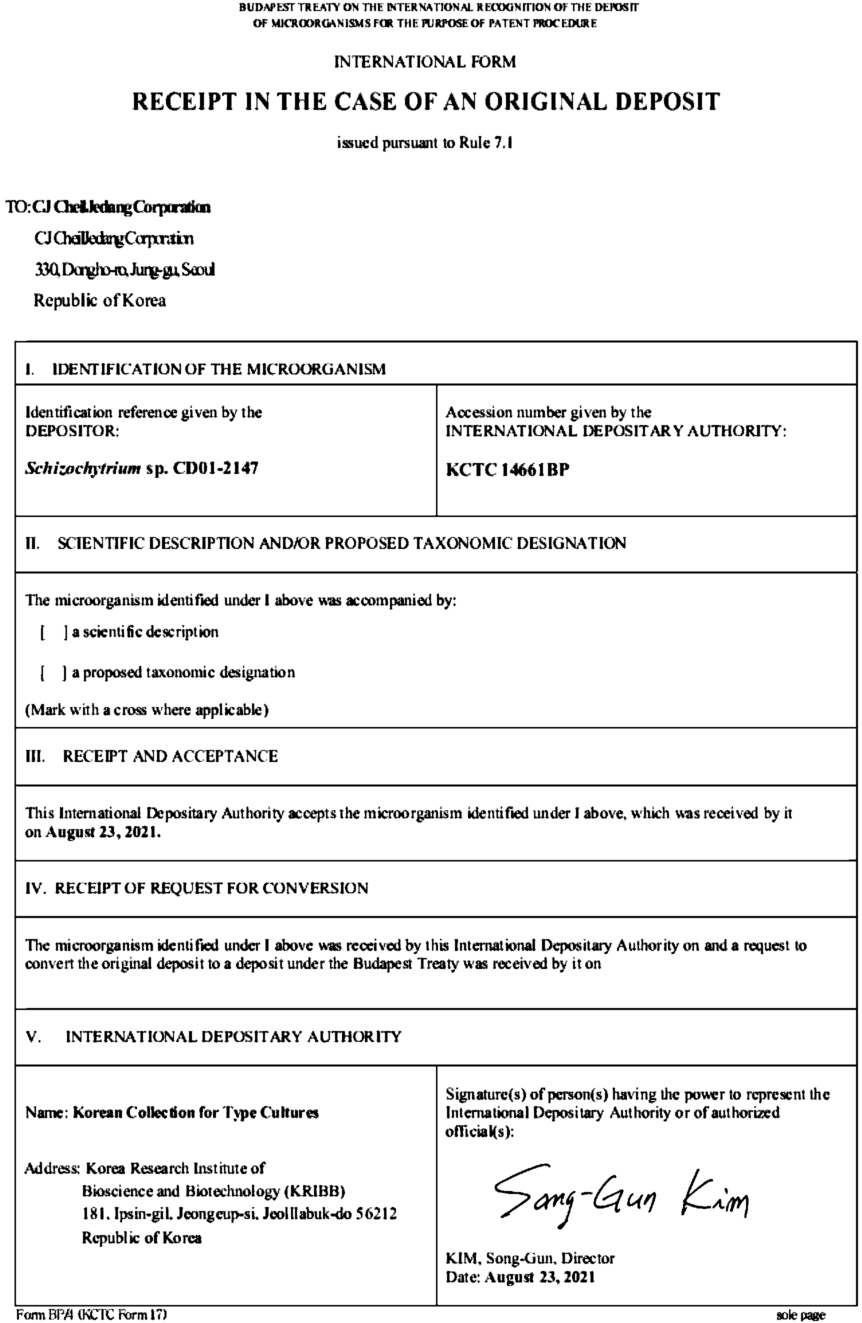
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: CJ CheilJedang Corporation
CJ CheilJedang Corporation
330, Dongho-ro, Jung-gu, Seoul
Republic of Korea

I. IDENTIFICATION OF THE MICROORGANISM
Identification reference given by the
DEPOSITOR:

Schizochytrium sp. CD01-2147 | Accession number given by the
INTERNATIONAL DEPOSITARY AUTHORITY:

KCTC 14661BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on August 23, 2021.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):


KIM, Song-Gun, Director
Date: August 23, 2021
Form BP/4 (KCTC Form 17) sole page

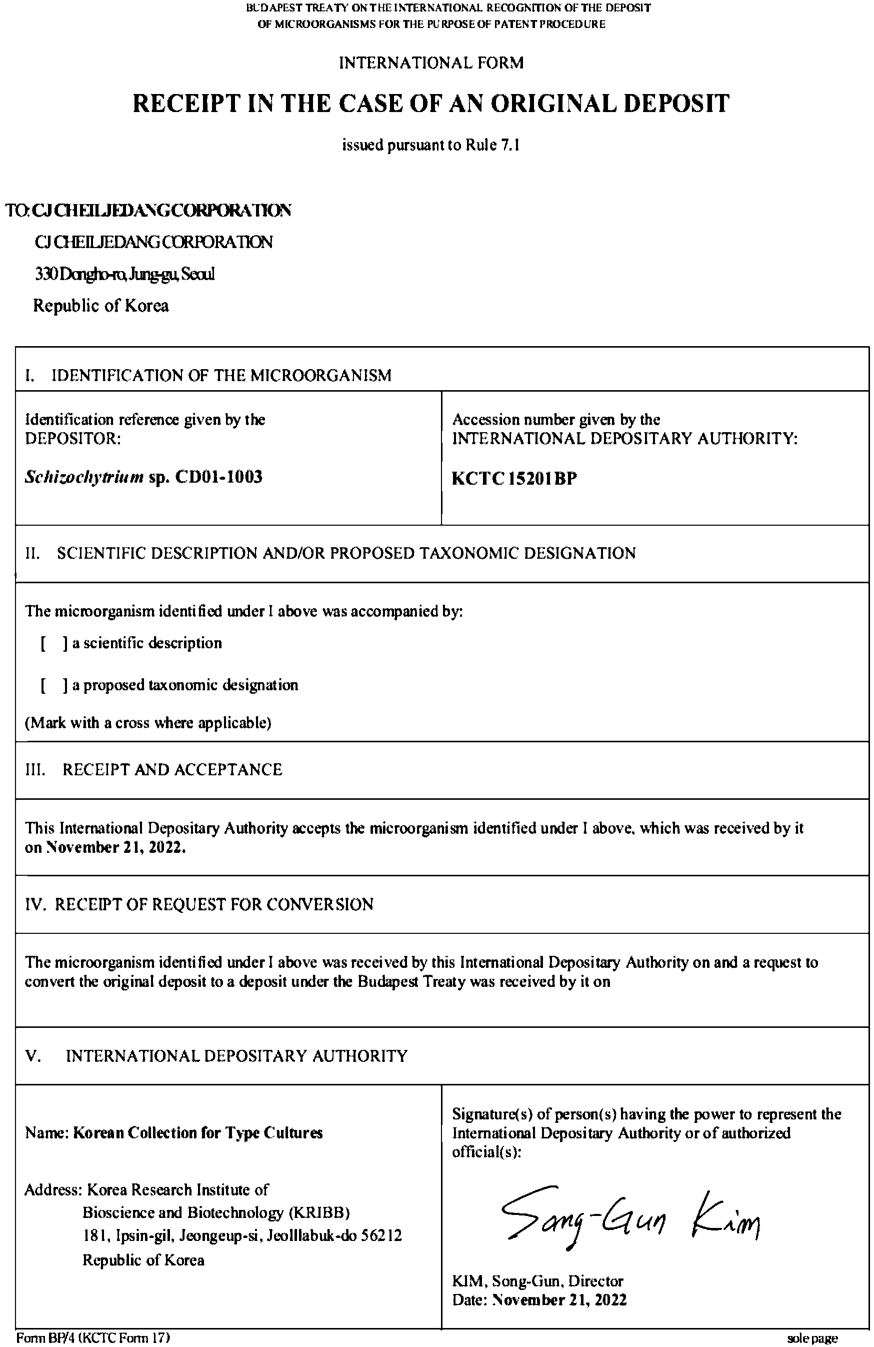
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: CJ CHEILJEDANG CORPORATION
CJ CHEILJEDANG CORPORATION
330 Dongho-ro, Jung-gu, Seoul
Republic of Korea

I. IDENTIFICATION OF THE MICROORGANISM
Identification reference given by the
DEPOSITOR:

Schizochytrium sp. CD01-1003 | Accession number given by the
INTERNATIONAL DEPOSITARY AUTHORITY:

KCTC 15201BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on November 21, 2022.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):


KIM, Song-Gun, Director
Date: November 21, 2022
Form BP/4 (KCTC Form 17) sole page

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: CJ CHEILJEDANG CORPORATION
CJ CHEILJEDANG CORPORATION
330 Dongho-ro, Jung-gu, Seoul
Republic of Korea

I. IDENTIFICATION OF THE MICROORGANISM
Identification reference given by the
DEPOSITOR:

Schizochytrium sp. CD02-8025 | Accession number given by the
INTERNATIONAL DEPOSITARY AUTHORITY:

KCTC 15246BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on December 15, 2022.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeolllabuk-do 56212
Republic of Korea | Signature(s) of person(s) having the power to represent the
International Depositary Authority or of authorized
official(s):

Song-Gun Kim

KIM, Song-Gun, Director
Date: December 15, 2022
Form BP/4 (KCTC Form 17) sole page

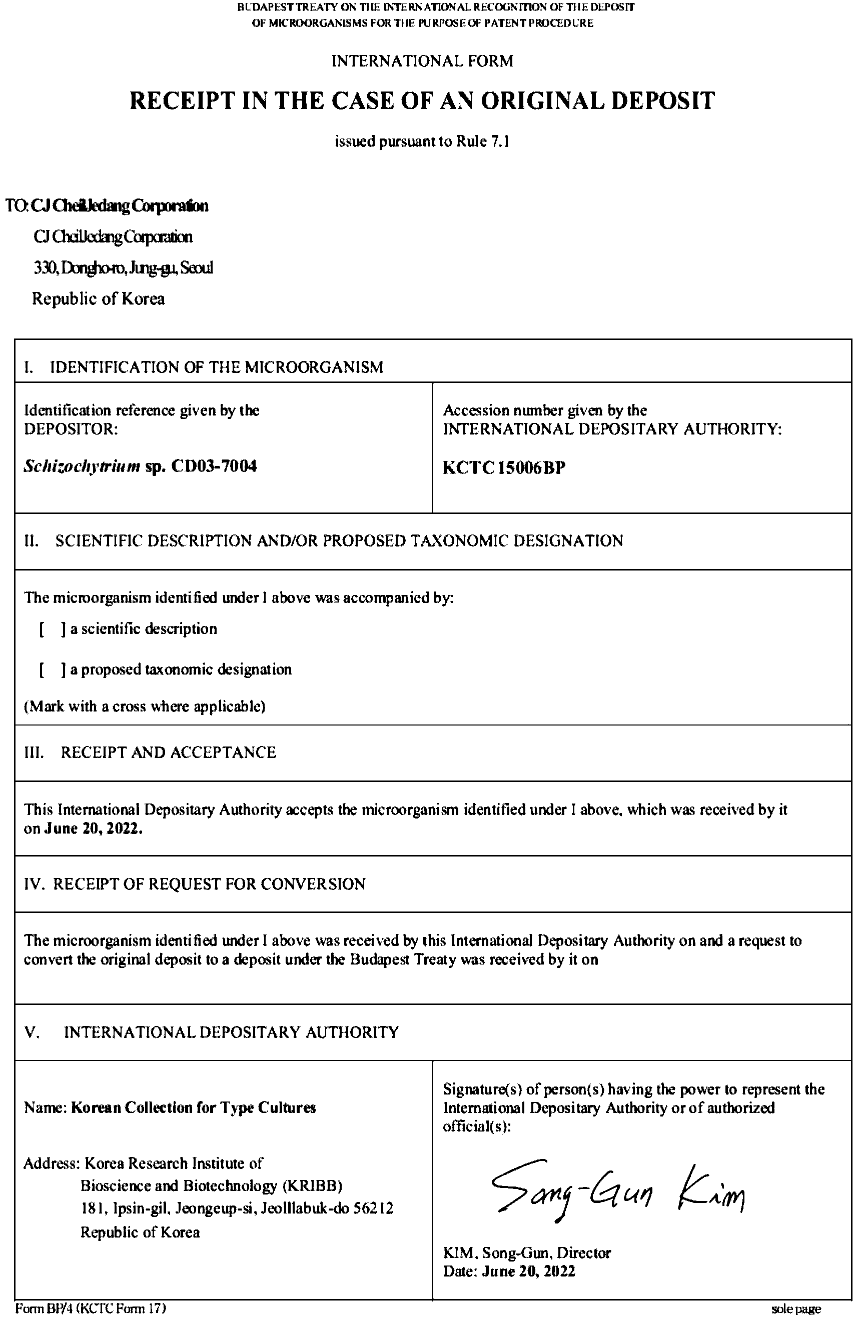
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: CJ CheilJedang Corporation
CJ CheilJedang Corporation
330, Dongho-ro, Jung-gu, Seoul
Republic of Korea

I. IDENTIFICATION OF THE MICROORGANISM
Identification reference given by the
DEPOSITOR: | Accession number given by the
INTERNATIONAL DEPOSITARY AUTHORITY:
Schizochytrium sp. CD03-7004 | KCTC 15006BP

II. SCIENTIFIC DESCRIPTION AND/OR PROPOSED TAXONOMIC DESIGNATION

The microorganism identified under I above was accompanied by:
[ ] a scientific description
[ ] a proposed taxonomic designation
(Mark with a cross where applicable)

III. RECEIPT AND ACCEPTANCE

This International Depositary Authority accepts the microorganism identified under I above, which was received by it
on June 20, 2022.

IV. RECEIPT OF REQUEST FOR CONVERSION

The microorganism identified under I above was received by this International Depositary Authority on and a request to
convert the original deposit to a deposit under the Budapest Treaty was received by it on

V. INTERNATIONAL DEPOSITARY AUTHORITY

Name: Korean Collection for Type Cultures | Signature(s) of person(s) having the power to represent the
International Depositary Authority or of authorized
official(s):
Address: Korea Research Institute of
Bioscience and Biotechnology (KRIBB)
181, Ipsin-gil, Jeongeup-si, Jeollabuk-do 56212
Republic of Korea |
KIM, Song-Gun, Director
Date: June 20, 2022

Form BP/4 (KCTC Form 17) sole page

## Claims

1. A DNA construct comprising a 5' homology arm, a target gene, and a 3' homology arm,
wherein the DNA construct is for inserting the target gene into a target region within the genome of a microorganism of genus *Schizochytrium,*
wherein the target region corresponds to a nucleic acid sequence of SEQ ID NO:1, SEQ ID NO: 2, or a nucleic acid sequence having at least 95% homology thereto in the genome of the microorganism of genus *Schizochytrium,*
wherein the 5' homology arm is capable of hybridizing to a region located within 100,000 bp upstream from the insertion site of the target gene within the target region,
and wherein the 3' homology arm is capable of hybridizing to a region located within 100,000 bp downstream from the insertion site of the target gene within the target region.

2. The DNA construct according to claim 1, wherein each of the 5' homology arm and the 3' homology arm independently has a size of 20 to 5,000 bp.

3. The DNA construct according to claim 1, wherein each of the 5' homology arm and the 3' homology arm independently has a size of 500 to 2,000 bp.

4. The DNA construct according to claim 1, wherein the target gene has a size of 30 to 100,000 bp.

5. The DNA construct according to claim 1, further comprising at least one selected from the group consisting of an expression regulatory sequence, a selection marker, and a reporter gene.

6. The DNA construct according to claim 1, wherein the insertion is achieved by at least one selected from the group consisting of homologous recombination (for example, homology-directed repair (HDR)), and non-homologous end joining (NHEJ).

7. The DNA construct according to any one of claim 1 to claim 6, wherein the microorganism of the genus *Schizochytrium* is at least one selected from the group consisting of *Schizochytrium limacinum, Schizochytrium mangrovei, Schizochytrium aggregatum,* and *Schizochytrium minutum.*

8. The DNA construct according to any one of claim 1 to claim 6, wherein the DNA construct is for delivering the target gene into the genome of the microorganism of the genus *Schizochytrium,* expressing the target gene in a microorganism of the genus *Schizochytrium,* or both thereof.

9. A composition for expressing a target gene in a microorganism of genus *Schizochytrium,* comprising the DNA construct of any one of claim 1 to claim 6.

10. A microorganism of genus *Schizochytrium* comprising the DNA construct of any one of claim 1 to claim 6.

11. The microorganism of genus *Schizochytrium* according to claim 10, wherein the microorganism of genus *Schizochytrium* is at least one selected from the group consisting of *Schizochytrium limacinum, Schizochytrium mangrovei, Schizochytrium aggregatum, and Schizochytrium minutum.*

12. A composition for producing a target product, comprising the DNA construct of any one of claim 1 to claim 6, a microorganism of genus *Schizochytrium* comprising the DNA construct, or a combination thereof.

13. A method for producing a microorganism of genus *Schizochytrium* for use in production of a target product, comprising a step of introducing the DNA construct of any one of claim 1 to claim 6 into the microorganism of genus *Schizochytrium.*

14. The method for producing a microorganism of genus *Schizochytrium* for use in production of a target product according to claim 13, wherein the microorganism of genus *Schizochytrium* is at least one species selected from the group consisting of *Schizochytrium limacinum, Schizochytrium mangrovei, Schizochytrium aggregatum,* and *Schizochytrium minutum.*

15. A method for producing a target product from a microorganism of genus *Schizochytrium,* comprising a step of culturing the microorganism of genus *Schizochytrium* comprising the DNA construct of any one of claim 1 to claim 6.

16. The method for producing a target product from a microorganism of genus *Schizochytrium* according to claim 15, further comprising a step of preparing the microorganism of genus *Schizochytrium* comprising the DNA construct, prior to the step of culturing.

17. The method for producing a target product from a microorganism of genus *Schizochytrium* according to claim 16, wherein the step of preparing the microorganism of genus *Schizochytrium* comprising the DNA construct, comprises a step of introducing the DNA construct into the microorganism of genus *Schizochytrium.*

18. The method for producing a target product from a microorganism of genus *Schizochytrium* according to claim 15, wherein the microorganism of genus *Schizochytrium* is at least one selected from the group consisting of *Schizochytrium limacinum, Schizochytrium mangrovei, Schizochytrium aggregatum,* and *Schizochytrium minutum.*
